# EUROPEAN PATENT SPECIFICATION

(11) **EP 3 344 033 B1**
(45) Date of publication and mention of the grant of the patent: **14.12.2022**
(21) Application number: 16778468.5
(22) Date of filing: 02.09.2016
(51) Int. Cl.: A01H 5/02, A01H 5/06, C08L 7/00, C08L 7/02

(54) **RUBBER PRODUCING TARAXACUM PLANT**
KAUTSCHUKPRODUZIERENDE TARAXACUM-PFLANZE
PLANTE TARAXACUM PRODUISANT DU CAOUTCHOUC

(30) Priority: 03.09.2015 NL 2015396
(43) Date of publication of application: 11.07.2018
(73) Proprietor: Lion-Flex B.V., 6515 AB Nijmegen (NL)
(72) Inventor: VAN DIJK, Peter Johannes, 6700 AE Wageningen (NL); SØRENSEN, Anker Preben, 6700 AE Wageningen (NL)
(74) Representative: Nederlandsch Octrooibureau
(86) International application number: PCT/NL2016/050613
(87) International publication number: WO 2017/039449

(56) References cited:
- Anonym: "Gib Gummi mit Kaukasischem Löwenzahn: die Pflanze ist potenzieller neuer Kautschuklieferant", Fachagentur Nachwachsende Rohstoffe , 15 July 2013 (2013-07-15), XP002764197, Retrieved from the Internet: URL:http://www.fnr.de/presse/pressemitteil ungen/archiv/archiv-nachricht/?tx_ttnews[y ear]=2013&tx_ttnews[month]=07&tx_ttnews[da y]=15&tx_ttnews[tt_news]=6411&cHash=909312 a21232bd9ada3259c0b3c2fde0 [retrieved on 2016-11-11]
- VAN BEILEN JAN B ET AL: "Guayule and Russian dandelion as alternative sources of natural rubber.", CRITICAL REVIEWS IN BIOTECHNOLOGY, vol. 27, no. 4, October 2007 (2007-10), pages 217-231, XP002764198, ISSN: 0738-8551
- VAN BEILEN JAN B ET AL: "Establishment of new crops for the production of natural rubber", TRENDS IN BIOTECHNOLOGY, vol. 25, no. 11, November 2007 (2007-11), pages 522-529, XP002764236, ISSN: 0167-7799
- Anonymous: "Kultevat, Keygene announce breakthrough in Russian dandelion latex.", RubberNews.com Kultevat natural grown quality, 26 March 2014 (2014-03-26), page 6PP, XP002764200, Retrieved from the Internet: URL:http://www.kultevat.com/news.html [retrieved on 2016-11-14]
- Anonymous: "EU-PEARLS Report Summary Project ID: 212827", European Commission , 18 July 2014 (2014-07-18), pages 1-21, XP002764201, Retrieved from the Internet: URL:http://cordis.europa.eu/result/rcn/144 740_en.pdf [retrieved on 2016-11-14]
- SCHMIDT THOMAS ET AL: "Molecular Cloning and Characterization of Rubber Biosynthetic Genes from Taraxacum koksaghyz", PLANT MOLECULAR BIOLOGY REPORTER, vol. 28, no. 2, June 2010 (2010-06), pages 277-284, XP002764202, ISSN: 0735-9640
- LAIBACH NATALIE ET AL: "Identification of a Taraxacum brevicorniculatum rubber elongation factor protein that is localized on rubber particles and promotes rubber biosynthesis.", THE PLANT JOURNAL : FOR CELL AND MOLECULAR BIOLOGY, vol. 82, no. 4, May 2015 (2015-05), pages 609-620, XP002764203, ISSN: 1365-313X
- EPPING JANINA ET AL: "A rubber transferase activator is necessary for natural rubber biosynthesis in dandelion", NATURE PLANTS, vol. 1, no. 5, April 2015 (2015-04), XP002764204,

## Description

### Technical field

The present invention relates to a vigorous and rubber producing *Taraxacum* plant, and a method for selecting such plant.

### Background of the invention

Natural rubber, an isoprene polymer, is an essential renewable material for more than 40,000 products essential to the construction industry (adhesives, sealants), pharmaceutical industry (gloves, tubing), and transportation industry (matting, tires). In many applications natural rubber cannot be replaced by synthetic rubbers (artificial elastomers are mainly synthesized from petroleum byproducts).

At the moment natural rubber is harvested exclusively from the rubber tree (*Hevea brasiliensis*) of which >90% is grown in South East Asia. However, worldwide natural rubber consumption is forecasted to increase significantly while production areas are under stress due to labor costs and preferences for palm oil production.

The Kazakh dandelion (*Taraxacum koksaghyz,* abbreviated TKS) also produces in its roots natural rubber of a very high quality, but it is a small plant leading to low yield. By contrast the common dandelion (*Taraxacum officinale,* abbreviated TO), is a vigorous plant, but produces no rubber.

Thus far, attempts to adequately combine vigour and rubber production into a single *Taraxacum* plant were unsuccessful (see e.g. Botanical Review, Vol. 11, No. 8, A Review of Literature on Taraxacum Kok-Saghyz Rod (Oct., 1945), pp. 417-461). It is an object of the present disclosure to solve at least this problem in the prior art.

### General definitions

In the following description and examples, a number of terms are used. In order to provide a clear and consistent understanding of the specification and claims, including the scope to be given such terms, the following definitions are provided. Unless otherwise defined herein, all technical and scientific terms used have the same meaning as commonly understood by one of ordinary skill in the art to which this disclosure belongs.

As used herein, the term "plant" or "plant material" can refer to plant cell, plant tissue or organs, plant protoplast, plant cell tissue culture from which plant(s) can be regenerated, plant calli, plant cell clumps, and plant cells that are intact in plants, part(s) of plant(s), such as embryos, pollen, ovules, fruit (e.g. harvested fruit), flowers, leaves, seeds, roots, root tips and the like. The root(s) is the organ of a plant that typically lies below the surface of the soil, and can be readily identified by a skilled person. The term "plant" can also refer to an entire plant, for example a *Taraxacum* plant, or roots thereof.

As used herein "rubber" refers to (natural) rubber, which comprises polymers of the organic compound isoprene, in particular cis-1,4-polyisoprene. Impurities of other organic compounds may be present (such as proteins, fatty acids, resins and inorganic materials), as well as water, but preferably the rubber comprises at least 95 wt.% isoprene polymers. In the rubber of the present disclosure, the (average) molecular weight of the polymer can be from 100,000 daltons to 1,000,000 daltons, preferably from 500,000 daltons to 1,000,000 daltons, from 800,000 to 1,000,000 daltons, or from 900,000 to 1,100,000 daltons, or even at least 1,000,000, 1,100,000, 1,200,000, 1,300,000, 1,400,000, 1,500,000, or at least 2,000,000 daltons. Forms of polyisoprene that are used as natural rubbers can be classified as elastomers. Natural rubber is used by many manufacturing companies for the production of rubber products, e.g. (car or airplane) tires.

As used herein *"Taraxacum"* refers to the genus of flowering plants in the family *Asteraceae* and consists of species commonly known as dandelion. *Taraxacum officinale* (TO), the common dandelion, is a flowering herbaceous perennial plant of the family. *Taraxacum koksaghyz* (TKS), commonly referred to as the Kazakh dandelion or Russian dandelion (or rubber dandelion), is a species of dandelion native to Kazakhstan that is notable for its production of high quality rubber but has low vigour. TKS can be found in nature in Kazakhstan, or can be obtained from the USDA seed bank. Diploid TO can for example be found in Switzerland, the Netherlands, or can also be obtained from the USDA seed bank. Of course, the *Taraxacum* plants according to the present disclosure are not limited to a particular source from where the original plants were obtained.

As used herein, "crossing", "backcrossing", "intercrossing", and "selecting" refers to steps in plant breeding. Plant breeding is the art of changing the traits of plants in order to produce desired characteristics. In other words, it is a process to choose individuals in a population that will contribute genetic material to the next generation (i.e. F1 being the generation resulting from the cross of the first set of parents (i.e. generation P), and F2/G2, G3, G4, G5, etc. being subsequent generations). In particular, such a process can be based both on natural or artificial phenomena or procedural steps. Selection criteria can be based on phenotypic or genomic characteristics, for instance, but not limited to, the presence, or degree of presence, of genes, gene expression, genetic markers, combinations of genes, quantitative trait loci, traits or combinations of traits. Backcrossing is a crossing of plants of a certain generation with one of its parents (or ancestors) or an individual genetically similar to its parent, in order to achieve offspring with a genetic identity which is closer to that of the parent or ancestor. Intercrossing refers to crossing two individuals that have a common/similar ancestry with one another. In the present disclosure, preferably, the crossing and/or backcrossing (and/or intercrossing) is done under optimal (greenhouse) conditions, e.g. 20-22 or 21 degrees Celsius at day time, 17-19 or 18 degrees Celsius at night time, normal humidity, and crossing steps are performed after 18-22 or 20 weeks from seedling.

As used herein, the term "marker" refers to a (DNA based) marker which can be used in a process, e.g. for plant breeding, wherein plants are screened for the presence and/or absence of one or more genetic and/or phenotypic markers. Examples of a genetic marker are a specific DNA sequence, AFLP (amplified fragment length polymorphism), microsatellite, RFLP (restriction fragment length polymorphism), STS (sequence tagged site), SNP (Single Nucleotide Polymorphism), SFP (Single Feature Polymorphism; see Borevitz et al. 2003, Genome Research 13: 513-523), SCAR (sequence characterized amplified region), CAPS markers (cleaved amplified polymorphic sequence) and the like. A KASP assay can be used to detect the presence or absence of a DNA sequence (marker), and/or to detect if a plant is heterozygous or homozygous for the marker (see also "SNP genotyping: the KASP assay, in Crop Breeding: Methods and Protocols, Springer 2014, Volume 1145). Also other method may be used to screen for the presence/absence of a certain DNA sequence (marker), for example regular PCR methods. Of course, a DNA sequence (marker) can also be detected via sequencing.

As used herein, the term "sequencing" refers to determining the order of nucleotides (base sequences) in a nucleic acid sample (DNA sample), e.g. obtained from a plant. Many techniques are available such as Sanger sequencing and High Throughput Sequencing technologies (HTS). Sanger sequencing may involve sequencing via detection through (capillary) electrophoresis, in which up to 384 capillaries may be sequence analysed in one run. High throughput sequencing involves the parallel sequencing of thousands or millions or more sequences at once. HTS can be defined as Next Generation sequencing, i.e. techniques based on solid phase pyrosequencing or as Next-Next Generation sequencing based on single nucleotide real time sequencing (SMRT). HTS technologies are available such as offered by Roche, Illumina and Applied Biosystems (Life Technologies). Further high throughput sequencing technologies are described by and/or available from Helicos, Pacific Biosciences, Complete Genomics, Ion Torrent Systems, Oxford Nanopore Technologies, Nabsys, ZS Genetics, GnuBio.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one". It is further understood that, when referring to "sequences" herein, generally the actual physical molecules with a certain sequence of subunits (e.g. bases) are referred to.

Methods of carrying out the conventional techniques used in methods of the present disclosure will be evident to the skilled worker, and are disclosed for example in Selection Methods in Plant Breeding (eds. Izak Bos, Peter Caligari; Springer Sciences + Business Media 2008; Progress in Plant Breeding (ed. G. E. Russel; Butterworth & Co, 2013); Quantitative Genetics, Genomics, and Plant Breeding (eds. Manjit S. Kang; KABI Publishing 2002); and Plant Breeding Methods (ed. Mahabal Ram; PHI Learning Private Limited, New Delhi, 2014).

### Description

The invention is set out in the appended set of claims.

The present disclosure relates to a method for providing a *Taraxacum* plant having
- a rubber content of at least 0.5 g, preferably at least 0.75, 1.0, 1.5, or even 2.0 g dry weight as can be determined preferably by Accelerated Solvent Extraction.

Also a *Taraxacum* plant is provided having
- a root dry weight of at least 5 g, preferably at least 7, 10, 20, 30, 40, or even 50 g;
- a rubber content in the roots of at least 3 wt.%, preferably at least 3.5, 4.0, 4.5, or even 5.0 wt.% dry weight with respect to total root dry weight as for example determined by Accelerated Solvent Extraction.

More specifically, the present disclosure relates to a method for providing and selecting *Taraxacum* plants, wherein the method comprises the following steps:
a) Providing *Taraxacum* plants having
   - a genome size of between 1000 - 1415 megabase, and/or
   - 60-99% *Taraxacum koksaghyz* (TKS) derived genes and 1-40% *Taraxacum officinale* (TO) derived genes;
b) Selecting *Taraxacum* plants for absence of the gene TO CPT;
c) Selecting *Taraxacum* plants for presence of at least three genes of TKS CPT2, TKS CPT3, TKS RTA, TKS SRPP5, and TKS REF;
d) selecting at most the top 40% *Taraxacum* plants with respect to plant size;
e) selecting at most the top 40%*Taraxacum* plants with respect to genome size.

The method combines TO vigour and TKS rubber production into a single *Taraxacum* plant.

This is achieved by introgression of vigour-related genetic elements of TO into the rubber producing genetic background of TKS, in order to obtain a vigorous and rubber producing *Taraxacum* plant.

For this, the first step a) relates to the provision of *Taraxacum* plants having a genome size of 1000 - 1415 megabase /1C (1C means the amount in a haploid nucleus, or half the amount in a diploid nucleus) and/or the provision of *Taraxacum* plants having 60-99% *Taraxacum koksaghyz* (TKS) derived genes and 1-40% *Taraxacum officinale* (TO) derived genes. The percentages of TO/TKS derived genes typically correlate with (and can be measured by) the genome size of the *Taraxacum* plant. TKS has a genome size of ~1420 megabase/1C (see e.g. Kirschner et al. 2013 (Genet Resour Crop Evol (2013) 60:455-471), while TO has a genome size of ~835 megabase/1C (unpublished data).

Although the breeding steps for obtaining such *Taraxacum* plants may be outsourced, it is also possible to obtain the plants by crossing and/or backcrossing (and/or intercrossing) *Taraxacum* plants, i.e. *Taraxacum koksaghyz* (TKS) plants and *Taraxacum officinale* (TO) plants, preferably over at least two generations. Therefore, step a) may be preceded by the provision of *Taraxacum koksaghyz* (TKS) plants and *Taraxacum officinale* (TO) plants, preferably followed by crossing and/or backcrossing (and/or intercrossing) TKS and TO. For both TKS and TO, diploid plants can be used for the first crossing step. After that, preferably, 1, 2, 3, or more backcrossing steps with TKS are performed.

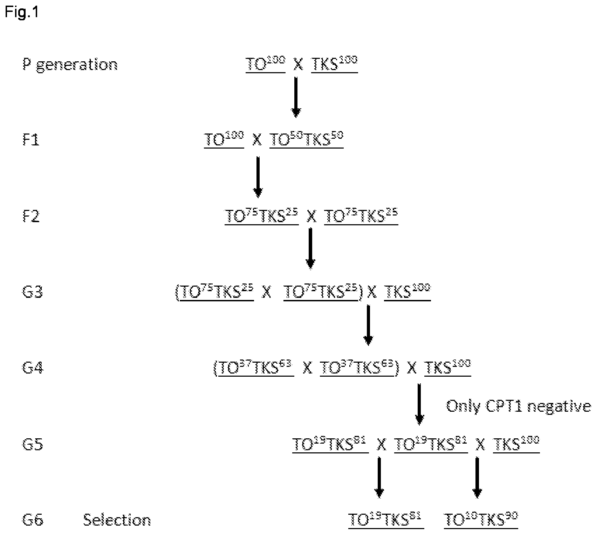

The skilled person can determine the genome size of a *Taraxacum* plant by flow cytometry as described for example by Tas and van Dijk (1999, Heredity 83: 707-714), and more preferably as described in the Example herein. The skilled person can also easily determine the percentages of TO/TKS derived genes based on the rule that progeny will have 50% of the genes of the first parent, and 50% of the genes of the second parent. This rule is visualized in Figure 1. In the first crossing step of Figure 1, TO (with 100% TO derived genes) is crossed with TKS (with 100% TKS derived genes), resulting in progeny with 50% TO derived genes and 50% TKS derived genes. These TO⁵⁰TKS⁵⁰ plants are then crossed with TO¹⁰⁰ plants, which results in TO(^{50/2+100/2})TKS (^{50/2}) = TO⁷⁵TKS²⁵, i.e. a Taraxacum plant with 75% TO derived genes, and 25% TKS derived genes. Since the percentages of TO/TKS derived genes typically are correlated with (and measured by) the genome size of the *Taraxacum* plant, it is possible, alternatively, that the percentages of TO/TKS derived genes are based on determining the genome size of the plant by flow cytometry. A genome size of ~835 megabase then means 100% TO, and each additional 5.85 megabase reduces the percentage of TO derived genes by 1%, and increases the percentage of TKS derived genes by 1%. So, if the genome size of a plant is determined to be 846.7 megabase, the plant can be seen as TO⁹⁸TKS². Accordingly, a genome size of ~1420 implies that the plant is 100% TKS.

Typically, in an optional breeding program, the first step will be to cross TO¹⁰⁰ with TKS¹⁰⁰ resulting in TO⁵⁰TKS⁵⁰. A second step can be backcrossing with TKS¹⁰⁰ resulting in TO²⁵TKS⁷⁵, which is already in the desired range as required by step a). However, it is preferred to perform multiple crossing steps. Applying more crossing, backcrossing and/or intercrossing steps has the advantage that more recombination events will occur, leading to more variation across plants, and a more diverse mixture of TO and TKS derived sections in the chromosomes of the plants provided in step a).

Therefore, it is preferred that crossing and/or backcrossing is applied over at least 1, 2, 3, 4, or 5 generations to arrive at the *Taraxacum* plants as required by step a), before proceeding to step b).

It will be clear to the skilled person that, when applying the present method, it is not required to perform the crossing, backcrossing, and/or intercrossing steps which lead to the provision of *Taraxacum* plants having a genome size of 1000 - 1415 megabase and/or having 60-99% *Taraxacum koksaghyz* (TKS) derived genes and 1-40% *Taraxacum officinale* (TO) derived genes. These steps are thus optional and may be outsourced such that the method can start with the provision of said plants.

In a preferred embodiment, step a) relates to the provision of *Taraxacum* plants a genome size of 1000 - 1415 megabase (/1C), or preferably a genome size of at least 850, 875, 900, 925, 950, 975, 1000, 1025, 1050, 1075, 1100, 1125, 1150, 1200, 1225, 1250, 1275, 1300, 1325, 1350, 1375, 1400 megabase (/1C) and/or at most 1100, 1125, 1150, 1200, 1225, 1250, 1275, 1300, 1325, 1350, 1375, 1400 megabase (/1C).

In another preferred embodiment, step a) relates to the provision of *Taraxacum* plants having at least 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, or 99% and/or at most 80, 81, 82, 83, 84, 85, 86, 87, 88, 89, 90, 91, 92, 93, 94, 95, 96, 97, 98 or 99% *Taraxacum koksaghyz* (TKS) derived genes and accordingly at most 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2 or 1% and/or at least 20, 19, 18, 17, 16, 15, 14, 13, 12, 11, 10, 9, 8, 7, 6, 5, 4, 3, 2, or 1% *Taraxacum officinale* (TO) derived genes. Alternatively, step a) relates to the provision of *Taraxacum* plants having between 60-99%, 65-95%, or 70-90% *Taraxacum koksaghyz* (TKS) derived genes and 1-40%, 5-35, or 10-30% *Taraxacum officinale* (TO) derived genes. The above-mentioned Taraxacum plants can be seen as hybrids of *Taraxacum koksaghyz* (TKS) and *Taraxacum officinale* (TO).

In step b), *Taraxacum* plants are selected for absence of the following *Taraxacum officinale* gene:
- CPT (TO CPT, *Taraxacum officinale* cis-prenyltransferase).

The skilled person can recognize TO CPT as it comprises (and can be detected by) the TO CPT marker as described in the Example under KASP assay.

It is preferred that step b) is performed after or before step a) of providing *Taraxacum* plants having a genome size of 1000 - 1415 megabase and/or having 60-99% *Taraxacum koksaghyz* (TKS) derived genes and 1-40% *Taraxacum officinale* (TO) derived genes. In this way, first selection *Taraxacum* plants can be provided.

In step c) *Taraxacum* plants are selected for presence of at least one, two, three, four, preferably at least five (or all) of the following *Taraxacum koksaghyz* genes:
- CPT2 (TKS CPT2, cis-prenyltransferase 2),
- CPT3 (TKS CPT3, cis-prenyltransferase 3),
- RTA (TKS RTA, rubber transferase activator),
- SRPP5 (TKS SRPP5, small rubber particle protein P5), and/or
- REF (TKS REF, rubber elongation factor).

The skilled person can recognize these genes based on that they comprise (and can be detected by) the respective marker as described in the Example under KASP assay.

In this way, second selection *Taraxacum* plants can be provided.

In a advantageous embodiment of step c), the plants are selected for homozygous presence of at least one, two, three, four, preferably at least five of the above listed *Taraxacum koksaghyz* genes.

Preferably in step c), *Taraxacum* plants are also selected for the (homozygous) presence of the whole TKS SRPP gene cluster, i.e. for TKS SRPP1-5: SRPP1 (small rubber particle protein P1), SRPP2 (small rubber particle protein P2), SRPP3 (small rubber particle protein P3), SRPP4 (small rubber particle protein P4), and SRPP5 (small rubber particle protein P5).

Further, it is preferred that the *Taraxacum* plants are additionally selected for (homozygous) presence of (the gene comprising) SEQ ID NO:11 as described herein in the Example. Preferably step c) is performed after step b) relating to selection against TO CPT and after or before step a) of providing *Taraxacum* plants having a genome size of 1000 - 1415 megabase (/1C) and/or having at least 60% *Taraxacum koksaghyz* (TKS) derived genes and at least 40% *Taraxacum officinale* (TO) derived genes.

The skilled person knows how presence/absence of the above-mentioned genes/sequences can be determined by identifying suitable markers that are associated with the presence or absence of said genes. For example, suitable primers can be designed for subsequent PCR detection, or DNA sequence markers of the genes can be detected by sequencing. A KASP assay is particularly useful for detecting (homozygous) presence/absence of the DNA sequence markers of the recited genes.

In step d) of the method, a selection is made for at most the top 40% *Taraxacum* plants with respect to plant size. Preferably, this step selects at most the top 35%, 30%, 25%, 20%, 15%, 10% *Taraxacum* plants with respect to plant size (thereby selecting for larger plants). Plant size can be measured in different ways, for example by determining the surface area of the longest leaf, the number of leaves, total plant weight, plant height (above the ground), or root weight. Preferably, plant size is determined based on the surface area of the longest leaf, or by multiplying said surface area with the number of leaves. Step d) is preferably performed after step a) (before step b), but more preferably after both steps b) and c). In this way, third selection *Taraxacum* plants can be provided.

In step e) of the method, a selection is made for at most the top 40%*Taraxacum* plants with respect to their genome size. Preferably, this step selects at most the top 35%, 30%, 25%, 20%, 15%, 10% *Taraxacum* plants with respect to their genome size (thereby selecting for plants with larger genomes). Genome size, e.g. in megabase, can be determined by flow cytometry, as well known to the skilled person, and as described by Tas and van Dijk (1999, Heredity 83: 707-714), and more preferably as described in the Example herein. Step e) is preferably performed after step a) (before step b), and more preferably after step d) of selecting on plant size. In this way, fourth selection *Taraxacum* plants can be provided.

Preferably step a) of the method provides at least 10, 20, 50, 100, 1000, 1500, or at least 2000 plants. The steps a)-e) may be performed in a different order than explicitly described, although the described order is preferred. Step b) can be optional. Step c) can also be optional. Also, step d) and/or step e) can be optional. The method as described above (or step a) thereof) preferably is not an essentially biological process for the production of plants and/or preferably does not involve crossing and subsequent selection of plants. Furthermore, it is preferred that in step b), c), d), and/or e), the selected plants are physically extracted out of the larger plant population with which the respective step starts. Simultaneously or alternatively, steps b), c), d), and/or e) can be computer-implemented steps, or performed *in silico.* The latter may be combined with step a) being optional.

Also provided is a *Taraxacum* plant which is obtainable or obtained by the method according to the present disclosure. Preferable, at least 2, 5, 10, 100, 500, 1000, 1500, or 2000 of such plants are provided. It shall be clear that a computer-readable medium comprising instructions for performing the present method is also foreseen.

The present disclosure further relates to a *Taraxacum* plant having
- a rubber content of at least 0.5 g, preferably at least 0.51, 0.52, 0.53, 0.54, 0.55, 0.56, 0.57, 0.58, 0.59, 0.6, 0.61, 0.62, 0.62, 0.63, 0.64, 0.65, 0.7, 0.75, 0.8, 0.85, 0.9, 0.95, or 1.0, 1.1, 1.2, 1.25, 1.3, 1.4, 1.50, 1.6, 1.75, 1.8, 1.9, 2.00, 2.1, 2.2, 2.25, 2.50, 2.75, or 3.0 g dry weight as can be determined preferably by Accelerated Solvent Extraction.

Also a *Taraxacum* plant is provided having
- a root dry weight of at least 5 g, preferably at least 5.5, 6.0, 6.5, 7.0, 7.5, 8.0, 9.0, 10, 11, 12, 13, 14, 15, 16, 17, 18, 19, 20, 21, 22, 23, 24, 25, 25, 27, 28, 29, 30, 31, 32, 33, 34, 35, 36, 37, 38, 39, or 40, 41, 42, 43, 44, 45, 46, 47, 48, 49, 50, 51, 52, 53, 54, 55, 56, 57, 58, 59, or 60 g; and/or
- a rubber content in the roots of at least 3 wt.%, preferably at least 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, or even 6.0 wt.% dry weight with respect to total root dry weight as for example determined by Accelerated Solvent Extraction.

Preferably, the plant additionally has at least one, two, three, four, five or all of the following genes/sequences:
- TKS CPT2 (preferably homozygous);
- TKS CPT3 (preferably homozygous);
- TKS RTA (preferably homozygous);
- TKS SRPP5 (preferably SRPP 1-5 and/or homozygous); and/or
- TKS REF (preferably homozygous); and
- optionally (the gene comprising) SEQ ID NO:11 (preferably homozygous) as described in the Example.

As described, the plant is preferably homozygous for at least one, two, three, four, five or six (or all) of these genes/marker sequences.

Further, the plant preferably has a genome size of at least 900, 910, 920, 930, 940, 950, 960, 970, 980, 990, or preferably at least 1000, or even at least 1050, 1100 1150, or at least 1200, 1250, 1275, 1300, 1325, 1350, 1375, 1400, 1410 megabase, as can be determined with flow cytometry (generally, in the determination, 1pg means 978 Mb). Preferably, pure TKS plant (taken to have 1420 megabase/1C) is used as (internal) reference, but also another (internal) reference having a known genome size can be used.

Alternatively or additionally, the plant has a genome size of at most 1200, 1250, 1275, 1300, 1325, 1350, 1375, 1400, 1410 megabase. Preferably the plant does not have TO CPT.

The above characteristics can be determined for a particular plant of the species *Taraxacum* at any time in its growth cycle, but preferably they are determined after at least 10, 11, 12, 13, 14, 15, 15, 20, 25, 30, 35, 40, 50, 100 weeks of growth under optimal (greenhouse) conditions starting from a seedling (e.g. at 21 degrees Celsius during day time, 18 degrees during night time, optionally humidity controlled).

Additionally or alternatively, the characteristics can be determined for a population of plants (e.g. 10, 20, 50, 100, 200, 500, 1000, or more plants) by calculating their average for each characteristic. Dry plant material can be obtained for example by oven drying at 70, 80, 90, or 100 degrees Celsius for 1, 2, or 3 hours.

The plant is preferably obtained/obtainable by the method, and/or for example at least 2, 5, 10, 100, 500, 1000, 1500, or 2000 of such plants are provided.

Rubber content can be determined by Accelerated Solvent Extraction (ASE) for example described as "traditional technique" in US 20060106183 A1. For example, the rubber may be recovered from the plant material (e.g. the roots), using parboiling, which coagulates the latex in the cells, followed by a milling step in a caustic solution to release the rubber. This traditional process then causes the milled bagasse to sink to the bottom of the processing vessel and allows resin to float to the surface for collection. More specifically, in this method, resins from plant materials are obtained by solvent extraction with polar solvents such as alcohols, ketones, and esters. A commonly used solvent is acetone. The resin is recovered from the solution by evaporating the solvent. The rubber from the shrub is generally extracted using hydrocarbon solvents such as hexane, cyclohexane or toluene.

Rubber content may be determined by ASE as follows
1. parboiling plant (root) material, or lyophilizing and grounding plant (root) material;
2. a milling step in a caustic solution to release the rubber, or subjecting the material to solvent (e.g. n-hexane with 2.5% EtOH) (1 min), then heating (to 80 °C) and pressurizing (1500 psi) the material (5 min),
3. (extracting the rubber with fresh solvent and) determining the weight of the rubber with respect to the weight of the starting material.

More preferably, the ASE method is performed as described in the Example.

Alternatively, rubber content may be determined by Accelerated Solvent Extraction with the recommended settings and variables as described in Pearson (Industrial Crops and Products 31 (2010) 469-475).

Preferably, rubber content is measured in the roots by taking at least two, three, four, five samples from the root parts of the plant, homogenizing them and then measuring rubber content. Alternatively, rubber content of each of the at least two, three, four, or five samples can be measured individually, and then their rubber content can be averaged.

The plant as can be provided by the present disclosure is preferably a non-bolter, i.e. does not bolt before cold induction. Bolting is the (premature) production of a flowering stem (or stems) on agricultural and horticultural crops before the crop is harvested. Non-bolters have the advantage that they will stay in a vegetative (growing) stage for a longer time, e.g. until after a winter passes. The skilled person knows how he/she can select for non-bolters. Further, preferably, the plant does not show summer dormancy. Summer dormancy is a yearly cycle that can occur in plants and which is caused by chemical changes within plant cells. It is stimulated by environmental condition with higher temperatures, relative dryness and longer days associated with late spring and summer, causing plant metabolism to come to a virtual standstill. The skilled person also knows how he/she can select for this characteristic.

The present disclosure also provides for a seed, cell or tissue culture, or root(s) derived from the plant according to the present disclosure, as well as for the use of the plant according to the present disclosure for rubber production. For the latter, the rubber is typically is extracted from the roots of the plant.

Accordingly, the present disclosure also provides for rubber obtainable for example by extraction from the (roots of the) plant according to the present disclosure.

The rubber according to the present disclosure may be distinguished from rubber obtained by prior art methods in that the rubber has different properties.

More specifically, the rubber can be characterized by
- an Mn of at least 300,000, 400,000, 500,000 dalton, preferably at least 510,000, 520,000, 530,000, 540,000, 550,000, 560,000, 570,000, 580,000, 590,000, 600,000, 700,000 or 800,000, 900,000 or even at least 1,000,000 dalton;
- an Mw of at least 700,000, 800,000, 900,000, preferably at least 910,000, 920,000, 930,000, 940,000, 950,000, 960,000, 970,000, 980,000, 990,000, 1,000,000, 1,100,000 or 1,200,000, 1,300,000 or even 1,400,000, or 1,500,000 or 1,600,000 or at least 1,700,000 dalton
- an Mz of at least 1,000,000, 1,100,000 or 1,200,000, 1,300,000 or even at least 1,400,000, or 1,500,000 or 1,600,000 or 1,700,000 dalton, 1,800,000, 1,900,000, or even at least 2,000,000, 2,100,000, 2,200,000, 2,300,000, 2,400,000, 2,500,000, 2,600,000, 2,700,000, 2,800,000, 2,900,000, or even 3,000,000, 3,100,000, 3,200,000, 3,300,000, 3,400,000, 3,500,000, 3,600,000, 3,700,000, 3,800,000, 3,900,000, or even 4,000,000 dalton; and/or
- a polydispersity of at most 10.0, 9.0, 8.0, 7.0, 6.0, 5.0, 4.0, 3.9, 3.8, 3.7, 3.6, 3.5, 3.4, 3.3, 3.2, 3.1, 3.0, 2.9, 2.8, 2.7, 2.6, 2.5, 2.4, 2.3, 2.2, 2.1, or 2.0.

Mn means the number-average molecular weight, Mw is the weight-average molecular weight, and Mz is the Z-average molecular weight. Polydispersity is the ratio Mw/Mn.

Additionally or alternatively, the rubber can be characterized by
- an Mn of at most 500,000 dalton, at most 510,000, 520,000, 530,000, 540,000, 550,000, 560,000, 570,000, 580,000, 590,000, 600,000, 700,000 or 800,000, 900,000 or even at most 1,000,000, 1,100,000, 1,200,000, 1,300,000, 1,400,000, 1,500,000, 1,600,000, 1,700,000, 1,800,000, 1,900,000 or at most 2,000,000 dalton;
- an Mw of at most 950,000, 960,000, 970,000, 980,000, 990,000, 1,000,000, 1,100,000 or 1,200,000, 1,300,000 or even at most 1,400,000, or 1,500,000 or 1,600,000 or 1,700,000, 1,800,000, 1,900,000, 2,000,000, or at most 2,500,000 dalton;
- an Mz of at most 1,000,000, 1,100,000 or 1,200,000, 1,300,000 or even 1,400,000, or at most 1,500,000 or 1,600,000 or 1,700,000 dalton, 1,800,000, 1,900,000, or even at most 2,000,000, 2,100,000, 2,200,000, 2,300,000, 2,400,000, 2,500,000, 2,600,000, 2,700,000, 2,800,000, 2,900,000, or even 3,000,000, 3,100,000, 3,200,000, 3,300,000, 3,400,000, 3,500,000, 3,600,000, 3,700,000, 3,800,000, 3,900,000, or even 4,000,000, 4,1000,000, 4,200,000, 4,300,000, 4,400,000, 4,500,000, 4,600,000, 4,700,000, 4,8000,000, 4,900,000, 5,000,000 dalton; and/or
- a polydispersity of at least 10.0, 9.0, 8.0, 7.0, 6.0, 5.0, 4.0, 3.0, 2.7, 2.6, 2.5, 2.4, 2.3, 2.2, 2.1, or 2.0, 1.9, 1.8, 1.7, 1.6, 1.5, 1.4, 1.3, 1.2, or 1.1 or at least 1.0.

Mn can be correlative with polymer colligative properties, e.g. freezing point depression. Mw may be correlated with properties such as melt viscosity. Mz may be correlated with properties such as toughness. Polydispersity characterizes the shape of the distribution: as the distribution decreases, the strength and toughness of the polymer generally increases.

Further explanation on Mn, Mw, Mz, polydispersity, and how they can be measured can be found for example in Chapter 17 "POLYMER MOLECULAR WEIGHT MEASUREMENT" of the Handbook of Polymer Synthesis, Characterization, and Processing, First Edition. Edited by Enrique Saldívar-Guerra and Eduardo Vivaldo-Lima. © 2013 John Wiley & Sons, Inc. Published 2013 by John Wiley & Sons, Inc. Preferably, the method as described in the Example is used for determining rubber quality and the above parameters.

A product comprising the rubber according to the present disclosure is also foreseen, wherein the product preferably is a tire, such as a car tire. As such, the present disclosure also provides for a car tire comprising the rubber according to the present disclosure.

In a specific embodiment, the present disclosure provides a method for producing a plant of the species *Taraxacum,* wherein the method comprises the following steps:
a)crossing *Taraxacum officinale* (TO) plants with *Taraxacum koksaghyz* (TKS) plants to provide generation F1 plants;
b)backcrossing the generation F1 plants with TO plants to provide generation F2 plants;
c) intercrossing the generation F2 plants to provide generation G3 plants;
d)intercrossing the generation G3 plants to provide generation G3' plants
e)backcrossing the generation G3' plants with TKS plants to provide generation G4 plants;
f) intercrossing the generation G4 plants to provide generation G4' plants;
g)backcrossing the generation G4' plants with TKS plants to provide generation G5 plants;
h)selecting generation G5 plants for absence of TO CPT to provide selected generation G5 plants;
i) optionally, intercrossing the selected generation G5 plants to provide generation G6-a plants, and/or backcrossing the selected generation G5 plants with TKS plants to provide generation G6-b plants;
j) selecting generation G6-a plants and/or selecting generation G6-b plants for presence of TKS CPT2, TKS CPT3, TKS RTA, TKS SRPP5 (preferably 1-5), TKS REF, and optionally SEQ ID NO:11 as described in the Example, to provide first selection generation G6 plants;
k) selecting at most the top 40% of the first selection generation G6 plants with respect to plant size (e.g. measured by surface area longest leaf, number of leafs, total plant weight, or plant height above the ground, or root weight) to provide second selection generation G6 plants;
l) selecting at most the top 40% of the second selection generation G6 plants *Taraxacum* plants with respect to genome size to provide third selection generation G6 plants.

The above specific method is shown in Figure 1. Preferably step a), b), c), d), e), f), g), h), i), j), k) and/or l) provide at least 10, 20, 50, 100, 1000, 1500, or at least 2000 plants. The breeding process may further comprise 1, 2, 3, or more additional backcrossing steps with TKS. Step b) can be optional. Step c) can also be optional. Also, step d) can be optional. The skilled person can select steps having in mind the goal of the method to produce a plant of the genus *Taraxacum* with the specified characteristics. Likewise, step e) can be optional and/or step f) can be optional. In this regard, step g) can also be optional, while alternatively or additionally step h) can be optional. Step i) can also be optional, and/or step j) can be optional. Finally, step k) and/or step l) can be optional. In the methods according to the present disclosure, preferably TKS is always used as a pollen donor during crossing steps.

In step i), two populations of plants can be provided: (1) generation G6-a, and (2) generation G6-b plants. The two population may be combined or held separate in further steps of the method according to the present disclosure. Alternatively, either generation G6-a plants or generation G6-b plants are provided.

### Brief description of the Figures

Figure 1: . Introgression and selection scheme according to a specific embodiment of the present disclosure. Genotypes are underlined. The superscript indicates the percentage of TKS derived genes and TO derived genes, assuming absence of segregation distortion. In Generation 3, 4 and 5 plants are backcrossed to TKS and intercrossed (hybrid swarms). Intercrossing increases the recombination of the TKS and TO genomes, whereas backcrossing increases the proportion of the TKS genome. In G4, only plants without TOCPT1 were selected. Therefore successive generations also lack this gene. In the present disclosure, introgressed plants can be used as seed parents and not as pollen parents, or the introgressed plants can be used as pollen parents and not as seed parents.

### Example 1

The following Table shows the characteristics of two *Taraxacum* plants obtained by applying the method of the present disclosure.

| | **G4 plant 1** | **G4 plant 2** |
|---|---|---|
| Genome size as determined by flow cytometry (megabase) | 1193 | 1150 |
| CPT(1) | A | H |
| CPT2+3 | H | H |
| RTA | H | B |
| SRPP5 | H | A |
| REF | H | H |
| % dry weight rubber in the roots | ***4.27*** | ***4.84*** |
| Root dry weight (g) | 56.60 | 30.20 |
| Rubber yield (g) | ***2.42*** | ***1.46*** |
| Mn (g/mol) | 395640 | 583290 |
| Mw (g/mol) | 977430 | 1228100 |
| Mz (g/mol) | 1865100 | 3234800 |
| polydispersity | 2.47 | 2.11 |

| | | |
|---|---|---|
| (A=homozygous for the TKS derived gene, B=homozygous for the TO derived gene, H=heterozygous) | | |

### Material and methods

### Determining rubber content (Collins-Silva J, et al. 2012, Phytochemistry, 79:46-56)

A Dionex Accelerated Solvent Extractor (ASE) 200 Model (Sunnyvale, CA) was used to extract natural rubber from root tissue using hexane with 2.5% EtOH. Root tissue was weighted. Lyophilized and ground root tissue (approximately 0.1 g) from harvested plants and control plants was extracted in triplicate. Each sample was subjected to the following method: solvent (n-hexane with 2.5% EtOH) filling of ASE cell (1 min), heating and pressurizing of the cell to 80 °C and 1500 psi (5 min), 3 cycles of: a 5 min static extraction with flushing of fresh solvent, and a final purge with N₂. The final hexane extracts were dried down under N₂ and stored at 20 °C. Rubber content was determined gravimetrically.

### Rubber quality (Collins-Silva J, et al. 2012, Phytochemistry, 79:46-56)

For HPLC analyses, hexane extracts were resuspended in THF 2 ml overnight at RT. The root extracts were then analyzed by HPLC-GPC. The apparatus used for these analyses consisted of an HP 1100 HPLC (Agilent Technologies, Palo Alto, CA), one Phenogel 50 × 7.80 mm guard column and two Phenogel 10 l Linear columns (300 × 7.80 mm) (Phenomenex, Torrance, CA) connected in series, and a Sedex Model 75 Evaporative Light Scattering Detector (SEDERE, France). The samples were fractionated using an isocratic elution in toluene at 70 °C with a flow rate of 1 ml/minute. Chromatograms were generated and analyzed using ChemStation for LC Rev. A. 10.02 (1757) software (Agilent Technologies). Rubber polymer molecular weight was calculated from HPLC-GPC data using the Agilent GPC Data Analysis ChemStation Module (version B.01.01). Calibration curves for molecular weight analyses were generated from polystyrene standards (Polymer Laboratories, UK) with Mps (peak molecular weights) ranging from 1480 to 3114,000 daltons. Rubber was quantified by calculating rubber mass as a function of peak area using a standard curve generated from serial dilutions of known amounts of a synthetic polyisoprene (Kraton Polymers IR-401, Houston, TX). For each experiment, root extracts, molecular weight standards and mass quantification standards were analyzed in the same run to decrease variability.

*Flow cytometry protocol* (Tas and van Dijk 1999, Heredity 83: 707-714)

The genome size of plants was determined by measuring the nuclear DNA amount with a UV flow cytometer (PARTEC Ploidy Analyser, PARTEC GmbH, Műnster, Germany), using a modified protocol of Ulrich & Ulrich (1991 Protoplasma 165, 212-215). Fresh leaf (1 cm²) of the hybrid plant and a pure TKS plant (taken to have 1420 megabase/1C) was chopped together with a sharp razor blade in 1 mL nuclei extraction buffer (0.1 M citric acid containing 0.5% Tween 20). After filtration through a 50 µm filter (CellTrics, PARTEC), 1 mL of DAPI fluorescence solution (0.4 M sodium hydrogen phosphate, 0.2 M NaCl and 5 mg L⁻¹ DAPI (4',6-diamidino-2-phenylindole)) was added and samples were measured directly.

### KASP assay

Presence/absence of TO CPT, TKS CPT2, TKS CPT3, TKS RTA, TKS SRPP5, and TKS REF was determined by using a KASP assay with the following marker sequences (SNP in Bold). The KASP assay is known to the skilled person and for example described by Semagn et al (2013 Molecular Breeding. 33 (1): 1-14).
#1. Marker of TO CPT (CPT1_2_507T, SEQ ID NO:1) 
GAGCCCGTAAGGATTGCTGCTGAGAAGGCCATGGAAGCCACCGCTAAAAATTCAACCA
CGTATCTCCTCGTATGTGTTGCTTACACTTCTTCCCATGAAAT
#2. Marker of TKS CPT2 (CPT_3_192T, SEQ ID NO:2) 
CCWATCCCAGAACACATCGCCTTCATCCTCGATGGAAACCGAAGGTTCGCTAGGAAAT
GGAACCTCACAGAAGGCRCMGGCCACAAAACCGGYTTCCTAGC
 (W means A or T, Y means C or T, and M means A or C)
#3. Marker of TKS CPT2 and TKS CPT3 (CPT1_2_507C, SEQ ID NO:3) 
GAGCCCGTAAGGATTGCTGCTGAGAAGGCCATGGAAGCCACCGCTAAAAACTCAACCA
CGTATCTCCTCGTATGTGTTGCTTACACTTCTTCCCATGAAAT
 (if absence of TKS CPT2 is confirmed by marker 2, this marker detects specifically TKS CPT3; or, if absence of TKS CPT3 is confirmed by marker 4, this marker detects specifically TKS CPT2)
#4. Marker of TO CPT and TKS CPT3 (CPT_3_192C, SEQ ID NO:4) 
CCWATCCCAGAACACATCGCCTTCATCCTCGATGGAAACCGAAGGTTCGCCAGGAAAT
GGAACCTCACAGAAGGCRCMGGCCACAAAACCGGYTTCCTAGC
 (if absence of TO CPT is confirmed by the marker 1, this marker detects specifically TKS CPT3 or, if absence of TKS CPT3 is confirmed by marker 3, this marker detects specifically TO CPT)
#5. Marker of TKS RTA (RTA-1A, SEQ ID NO:5) 
CTTTGTTCGYGAGATCTTACGTGGAATTGAAARCTATCTTATAATAAATGAAATTGTGAAA
ACATATGAAGATCTGAATTTAAACAGAGTGAAATATCTTG
#6. Marker for TO RTA (RTA-1G, SEQ ID NO:6) 
CTTTGTTCGYGAGATCTTACGTGGAATTGAAARCTATCTTATAATAAATGGAATTGTGAAA
ACATATGAAGATCTGAATTTAAACAGAGTGAAATATCTTG
#7. Marker for TKS SRPP5 (SRPP5-2G, SRPP cluster, SEQ ID NO:7) 
TGTTAAATAAGTTATGAGCGATTGTTTGGATTCTTATGATMTTTTCATGAGCGACTGTGT
GCAAATTTCATCCAATAATCTATGTGAACGCTCTTACTTGT
#8. Marker for TKS REF (REF2C, SEQ ID NO:8) 
ATTATGCAGGCTAAGGTCGAGAATGGATCTACAAAATCTAAATTGTTAGACCTTTTGAAG
AAAATTTTCGCGGTATATTTGGCATTAGTCACGAGYGTGTT
#9. Marker for TO SRPP5 (SRPP5-2T, SRPP cluster, SEQ ID NO:9) 
TGTTAAATAAGTTATGAGCGATTGTTTGGATTCTTATGATMTTTTCATGATCGACTGTGTG
CAAATTTCATCCAATAATCTATGTGAACGCTCTTACTTGT
#10. Marker for TO REF (REF2G, SEQ ID NO:10) 
ATTATGCAGGCTAAGGTCGAGAATGGATCTACAAAATCTAAATTGTTAGAGCTTTTGAAG
AAAATTTTCGCGGTATATTTGGCATTAGTCACGAGYGTGTT
#11. Additional TKS Marker (SEQ ID NO:11) 
CGACTTTATGCTCCGGTGAGGGTGGCGGCGGAGAAGGCGATGGAGGCCACAGCTAAG
AACACGAAGACTTATCTTTTGGTGTGTGTGGCGTATACKTCRTC
#12 Additional TO marker (SEQ ID NO:12)

CGACTTTATGCTCCGGTGAGGGTGGCGGCGGAGAAGGCGATGGAGGCCACGGCTAAG
AACACGAAGACTTATCTTTTGGTGTGTGTGGCGTATACKTCRTC

Presence of a marker in a plant sample indicates presence of the respective gene in the plant from which the sample was obtained. Further, the KASP assay allows to determine whether the plant is heterozygous or homozygous for the respective gene, based on the detected signal, as well known to the skilled person.

## Claims

1. Method for selecting hybrid *Taraxacum* plants, wherein the method comprises the following steps:
a) Providing hybrid *Taraxacum* plants having a genome size of 1000 - 1415 megabase;
b) selecting hybrid *Taraxacum* plants for absence of the gene *Taraxacum officinale* (TO) CPT;
c) selecting hybrid *Taraxacum* plants for presence of at least three genes selected from the group consisting of *Taraxacum koksaghyz* (TKS) CPT2 (cis-prenyltransferase 2), TKS CPT3 (cis-prenyltransferase 3), TKS RTA (rubber transferase activator), TKS SRPP5 (small rubber particle protein 5), and TKS REF (rubber elongation factor);
d) selecting at most the top 40% hybrid *Taraxacum* plants having the largest plant size; and
e) selecting at most the top 40% hybrid *Taraxacum* plants having the largest genome size, wherein the hybrid *Taraxacum* plants are a hybrid of *Taraxacum officinale* and *Taraxacum koksaghyz.*

2. Method according to claim 1, wherein in step a) *Taraxacum* plants are provided having a genome size of 1100 - 1415 megabase and/or having 60-99% *Taraxacum koksaghyz* (TKS) derived genes and 1-40% *Taraxacum officinale* (TO) derived genes.

3. Method according to claim 1 or claim 2, wherein
in step b) the *Taraxacum* plant is selected for the absence of a TO CPT specific genetic marker, wherein preferably the marker has at least one of SEQ ID NO: 1 and SEQ ID NO: 4; and/or
in step c) the *Taraxacum* plant is selected for the presence of at least three genetic markers, wherein each marker is specific for a gene selected from the group consisting of TKS CPT2, TKS CPT3, TKS RTA, TKS SRPP5, and TKS REF, wherein preferably:
i) the TKS CPT2 marker has at least one of SEQ ID NO: 2 and SEQ ID NO: 3;
ii) the TKS CPT3 marker has at least one of SEQ ID NO: 3 and SEQ ID NO: 4;
iii) the TKS RTA marker has SEQ ID NO: 5;
iv) the TKS SRPP5 marker has SEQ ID NO: 7; and
v) the TKS REF marker has SEQ ID NO: 8.

4. Method according to any one of the previous claims, wherein step c) selects for presence of at least four, preferably at least five genes selected from the group consisting of TKS CPT2, TKS CPT3, TKS RTA, TKS SRPP5, and TKS REF.

5. Method according to any one of the previous claims, wherein step c) selects for homozygous presence of one or more genes selected from the group consisting of TKS CPT2, TKS CPT3, TKS RTA, TKS SRPP5, and TKS REF.

6. Computer-readable medium comprising instructions for performing the method according to any one of the previous claims.

7. A hybrid *Taraxacum* plant, wherein the hybrid plant lacks the gene *Taraxacum officinale* (TO) CPT and comprises at least three genes selected from the group consisting of *Taraxacum koksaghyz* (TKS) CPT2, TKS CPT3, TKS RTA, TKS SRPP5, and TKS REF, and wherein the hybrid *Taraxacum* plant is a hybrid of *Taraxacum officinale* and *Taraxacum koksaghyz.*

8. *Taraxacum* plant according to claim 7 having
- a rubber content of at least 0.5 g, preferably at least 0.75, 1.0, 1.25, 1.50, 1.75, or at least 2.00 g dry weight as determined by Accelerated Solvent Extraction.

9. *Taraxacum* plant according to claim 7 or 8, having
- a root dry weight of at least 5 g, preferably at least 6, 7, 8, 9, or 10 g; and
- a rubber content of at least 3 wt.% preferably at least 3.5, 4.0, 4.5, or 5.0 wt.% dry weight with respect to total root dry weight as determined by Accelerated Solvent Extraction.

10. *Taraxacum* plant according to any one of claims 7-9 comprising the genes TKS CPT2, TKS CPT3, TKS RTA, TKS SRPP5, and TKS REF.

11. *Taraxacum* plant according to any one of claims 7-10 having
- a genome size of at least 1000 megabase, preferably at least 1100 megabase.

12. *Taraxacum* plant according to any one of claims 7-11, wherein the plant is homozygous for one or more genes selected from the group consisting of TKS CPT2, TKS CPT3, TKS RTA, TKS SRPP5, and TKS REF.

13. Use of a *Taraxacum* plant according to any one of claims 7-12 for rubber production.

14. Method for the manufacture of rubber, comprising a step of extracting the rubber from the plant according any one of claims 7-12.

15. Method according to claim 14, wherein the rubber is **characterized by**
- an Mn of at least 500,000 g/mole;
- an Mw of at least 1,000,000 g/mole;
- an Mz of at least 2,500,000 g/mole; and
- a polydispersity of between 1.0 and 5.0.

## Patentansprüche

1. Verfahren für die Auswahl von hybriden *Taraxacum-Pflanzen,* wobei das Verfahren die folgenden Schritte umfasst:
a) Bereitstellen von hybriden *Taraxacum-Pflanzen* mit einer Genomgröße von 1000 - 1415 Megabasen;
b) Auswählen von hybriden *Taraxacum-Pflanzen* auf die Abwesenheit des Gens *Taraxacum officinale* (TO) CPT;
c) Auswählen von hybriden *Taraxacum-Pflanzen* auf die Anwesenheit von mindestens drei Genen ausgewählt aus der Gruppe bestehend aus *Taraxacum koksaghyz* (TKS) CPT2 (cis-Prenyltransferase 2), TKS CPT3 (cis-Prenyltransferase 3), TKS RTA (Kautschuktransferaseaktivator), TKS SRPP5 (kleines Kautschukteilchenprotein 5) und TKS REF (Kautschukverlängerungsfaktor);
d) Auswählen von höchstens der besten 40% der hybriden *Taraxacum-*Pflanzen mit der größten Pflanzengröße; und
e) Auswählen von höchstens der besten 40% der hybriden *Taraxacum-*Pflanzen mit der größten Genomgröße, wobei die hybriden *Taraxacum-Pflanzen* ein Hybrid von *Taraxacum officinale* und *Taraxacum koksaghyz* sind.

2. Verfahren nach Anspruch 1, wobei in Schritt a) *Taraxacum-Pflanzen* bereitgestellt werden mit einer Genomgröße von 1100 bis 1415 Megabasen und/oder mit 60-99% von *Taraxacum koksaghyz* (TKS) stammenden Genen und 1-40% von *Taraxacum officinale* (TO) stammenden Genen.

3. Verfahren nach Anspruch 1 oder Anspruch 2, wobei
in Schritt b) die *Taraxacum-Pflanze* ausgewählt wird für die Abwesenheit eines TO CPT spezifischen genetischen Markers, wobei bevorzugt der Marker mindestens eine von SEQ ID NO: 1 und SEQ ID NO: 4 aufweist; und/oder
in Schritt c) die *Taraxacum-Pflanze* ausgewählt wird für die Anwesenheit von mindestens drei genetischen Markern, wobei jeder Marker spezifisch für ein Gen ausgewählt aus der Gruppe bestehend aus TKS CPT2, TKS CPT3, TKS RTA, TKS SRPP5 und TKS REF ist, wobei bevorzugt:
i) der TKS CPT2 Marker mindestens eine von SEQ ID NO: 2 und SEQ ID NO: 3 aufweist;
ii) der TKS CPT3 Marker mindestens eine von SEQ ID NO: 3 und SEQ ID NO: 4 aufweist;
iii) der TKS RTA Marker SEQ ID NO: 5 aufweist;
iv) der TKS SRPP5 Marker SEQ ID NO: 7 aufweist; und
v) der TKS REF Marker SEQ ID NO: 8 aufweist.

4. Verfahren nach einem der vorhergehenden Ansprüche, wobei Schritt c) auswählt für die Anwesenheit von mindestens vier, bevorzugt mindestens fünf Genen ausgewählt aus der Gruppe bestehend aus TKS CPT2, TKS CPT3, TKS RTA, TKS SRPP5 und TKS REF.

5. Verfahren nach einem der vorhergehenden Ansprüche, wobei Schritt c) auswählt für die homozygote Anwesenheit von einem oder mehreren Genen ausgewählt aus der Gruppe bestehend aus TKS CPT2, TKS CPT3, TKS RTA, TKS SRPP5 und TKS REF.

6. Computerlesbares Medium umfassend die Anweisungen für die Durchführung des Verfahrens nach einem der vorhergehenden Ansprüche.

7. Hybride *Taraxacum-Pflanze,* wobei die hybride Pflanze das Gen *Taraxacum officinale* (TO) CPT nicht aufweist und mindestens drei Gene ausgewählt aus der Gruppe bestehend aus *Taraxacum koksaghyz* (TKS) CPT2, TKS CPT3, TKS RTA, TKS SRPP5 und TKS REF umfasst, und wobei die hybride *Taraxacum-Pflanze* ein Hybrid von *Taraxacum officinale* und *Taraxacum koksaghyz* ist.

8. *Taraxacum-Pflanze* nach Anspruch 7, aufweisend
- einen Kautschukgehalt von mindestens 0,5 g, bevorzugt mindestens 0,75, 1,0, 1,25, 1,50, 1,75 oder mindestens 2,0 g Trockengewicht bestimmt durch beschleunigte Lösungsmittelextraktion.

9. *Taraxacum-Pflanze* nach Anspruch 7 oder 8, aufweisend
- ein Wurzeltrockengewicht von mindestens 5 g, bevorzugt mindestens 6, 7, 8, 9 oder 10 g; und
- einen Kautschukgehalt von mindestens 3 Gew.-%, bevorzugt mindestens 3,5, 4,0, 4,5 oder 5,0 Gew.-% Trockengewicht mit Bezug auf das Gesamtwurzeltrockengewicht bestimmt durch beschleunigte Lösungsmittelextraktion.

10. *Taraxacum-Pflanze* nach einem der Ansprüche 7 bis 9, umfassend die Gene TKS CPT2, TKS CPT3, TKS RTA, TKS SRPP5 und TKS REF.

11. *Taraxacum-Pflanze* nach einem der Ansprüche 7 bis 10, aufweisend
- eine Genomgröße von mindestens 1000 Megabasen, bevorzugt mindestens 1100 Megabasen.

12. *Taraxacum-Pflanze* nach einem der Ansprüche 7 bis 11, wobei die Pflanze homozygot für ein oder mehrere Gene ausgewählt aus der Gruppe bestehend aus TKS CPT2, TKS CPT3, TKS RTA, TKS SRPP5 und TKS REF ist.

13. Verwendung einer *Taraxacum-Pflanze* nach einem der Ansprüche 7 bis 12 für die Kautschukherstellung.

14. Verfahren für die Herstellung von Kautschuk, umfassend den Schritt der Extraktion des Kautschuks aus der Pflanze nach einem der Ansprüche 7 bis 12.

15. Verfahren nach Anspruch 14, wobei der Kautschuk **gekennzeichnet ist, durch**
ein Mn von mindestens 500 000 g/mol;
ein Mw von mindestens 1 000 000 g/mol;
ein Mz von mindestens 2 500 000 g/mol; und
eine Polydispersität zwischen 1,0 und 5,0.

## Revendications

1. - Procédé pour sélectionner des plantes hybrides de *Taraxacum,* dans lequel le procédé comprend les étapes suivantes :
a) fournir des plantes hybrides de *Taraxacum* ayant une taille de génome allant de 1000 à 1415 mégabases ;
b) sélectionner des plantes hybrides de *Taraxacum* pour l'absence du gène *Taraxacum officinale* (TO) CPT ;
c) sélectionner des plantes hybrides de *Taraxacum* pour la présence d'au moins trois gènes choisis dans le groupe consistant en *Taraxacum koksaghyz* (TKS) CPT2 (cis-prenyltransferase 2), TKS CPT3 (cis-prenyltransferase 3), TKS RTA (rubber transferase activator, activateur de la transférase du caoutchouc), TKS SRPP5 (small rubber particle protein 5, protéine 5 à petite particule de caoutchouc) et TKS REF (rubber elongation factor, facteur d'élongation du caoutchouc) ;
d) sélectionner au maximum les 40 % de plantes hybrides de *Taraxacum* ayant la plus grande taille de plante ; et
e) sélectionner au maximum les 40 % de plantes hybrides de *Taraxacum* ayant la plus grande taille de génome,
dans lequel les plantes hybrides de *Taraxacum* sont un hybride de *Taraxacum officinale* et *Taraxacum koksaghyz.*

2. - Procédé selon la revendication 1, dans lequel, dans l'étape a), des plantes de *Taraxacum* sont fournies, lesquelles ayant une taille de génome allant de 1100 à 1415 mégabases et/ou ayant 60 à 99 % de gènes dérivés de *Taraxacum koksaghyz* (TKS) et 1 à 40 % de gènes dérivés de *Taraxacum officinale* (TO).

3. - Procédé selon la revendication 1 ou la revendication 2, dans lequel :
dans l'étape b), la plante de *Taraxacum* est sélectionnée pour l'absence d'un marqueur génétique spécifique de TO CPT, dans lequel le marqueur a de préférence au moins l'une de SEQ ID NO : 1 et SEQ ID NO : 4 ; et/ou
dans l'étape c), la plante de *Taraxacum* est sélectionnée pour la présence d'au moins trois marqueurs génétiques, dans lequel chaque marqueur est spécifique d'un gène choisi dans le groupe consistant en TKS CPT2, TKS CPT3, TKS RTA, TKT SRPP5 et TKS REF, dans lequel de préférence :
i) le marqueur TKS CPT2 a au moins l'une de SEQ ID NO : 2 et SEQ ID NO : 3 ;
ii) le marqueur TKS CPT3 a au moins l'une de SEQ ID NO : 3 et SEQ ID NO : 4 ;
iii) le marqueur TKS RTA a SEQ ID NO : 5 ;
iv) le marqueur TKS SRPP5 a SEQ ID NO : 7 ; et
v) le marqueur TKS REF a SEQ ID NO : 8.

4. - Procédé selon l'une quelconque des revendications précédentes, dans lequel l'étape c) sélectionne la présence d'au moins quatre, de préférence d'au moins cinq, gènes choisis dans le groupe consistant en TKS CPT2, TKS CPT3, TKS RTA, TKS SRPP5 et TKS REF.

5. - Procédé selon l'une quelconque des revendications précédentes, dans lequel l'étape c) sélectionne la présence homozygote d'un ou plusieurs gènes choisis dans le groupe consistant en TKS CPT2, TKS CPT3, TKS RTA, TKS SRPP5 et TKS REF.

6. - Support lisible par ordinateur comprenant des instructions pour réaliser le procédé selon l'une quelconque des revendications précédentes.

7. - Plante hybride de *Taraxacum,* dans laquelle la plante hybride est dépourvue du gène *Taraxacum officinale* (TO) CPT et comprend au moins trois gènes choisis dans le groupe consistant en *Taraxacum koksaghyz* (TKS) CPT2, TKS CPT3, TKS RTA, TKS SRPP5 et TKS REF, et dans laquelle la plante hybride de *Taraxacum* est un hybride de *Taraxacum officinale* et *Taraxacum koksaghyz.*

8. - Plante de *Taraxacum* selon la revendication 7 ayant :
- une teneur en caoutchouc d'au moins 0,5 g, de préférence d'au moins 0,75, 1,0, 1,25, 1,50, 1,75, ou d'au moins 2,00 g de poids sec telle que déterminée par extraction accélérée par solvant.

9. - Plante de *Taraxacum* selon l'une des revendications 7 ou 8, ayant :
- un poids sec des racines d'au moins 5 g, de préférence d'au moins 6, 7, 8, 9 ou 10 g ; et
- une teneur en caoutchouc d'au moins 3 % en poids, de préférence d'au moins 3,5, 4,0, 4,5 ou 5,0 % en poids de poids sec par rapport au poids sec total des racines, telle que déterminée par extraction accélérée par solvant.

10. - Plante de *Taraxacum* selon l'une quelconque des revendications 7 à 9 comprenant les gènes TKS CPT2, TKS CPT3, TKS RTA, TKS SRPP5 et TKS REF.

11. - Plante de *Taraxacum* selon l'une quelconque des revendications 7 à 10 ayant :
- une taille de génome d'au moins 1000 mégabases, de préférence d'au moins 1100 mégabases.

12. - Plante de *Taraxacum* selon l'une quelconque des revendications 7 à 11, dans laquelle la plante est homozygote pour un ou plusieurs gènes choisis dans le groupe consistant en TKS CPT2, TKS CPT3, TKS RTA, TKS SRPP5 et TKS REF.

13. - Utilisation d'une plante de *Taraxacum* selon l'une quelconque des revendications 7 à 12 pour la production de caoutchouc.

14. - Procédé pour la fabrication de caoutchouc, comprenant une étape d'extraction du caoutchouc à partir de la plante selon l'une quelconque des revendications 7 à 12.

15. - Procédé selon la revendication 14, dans lequel le caoutchouc est **caractérisé par** :
- une Mn d'au moins 500 000 g/mole ;
- une Mw d'au moins 1 000 000 g/mole ;
- une Mz d'au moins 2 500 000 g/mole ; et
- une polydispersité comprise entre 1,0 et 5,0.
